# EUROPÄISCHE PATENTANMELDUNG

(11) **EP 1 795 169 A1**
(43) Veröffentlichungstag der Anmeldung: **13.06.2007**
(21) Anmeldenummer: 05026568.5
(22) Anmeldetag: 06.12.2005
(51) Int. Cl.: A61J 1/00, A61M 5/14

(54) **Vorrichtung zur Verabreichung enteraler Ernährung**

(71) Anmelder: Fritzmeier, Hildegard, 91710 Gunzenhausen (DE); Gemü GmbH, 6343 Rotkreuz (CH)
(72) Erfinder: Rominger, Lars, Dipl. Ing., 6313 Edlibach (CH); Fritzmeier, Hildegard, 91710 Gunzenhausen (DE)
(74) Vertreter: Zech, Stefan Markus

(57) **Zusammenfassung**

Die Erfindung betrifft eine Vorrichtung zur Verabreichung enteraler Ernährung umfassend ein erstes Reservoir (11) sowie ein zweites Reservoir (12), die jeweils über eine Absperrverteilervorrichtung (13) mit einem zum Patienten führenden Anschluss (26) in Verbindung stehen. Dabei ist das erste Reservoir (11) zur Befüllung mit enteraler Ernährung und das zweite Reservoir (12) zur Befüllung mit Flüssigkeit, insbesondere Tee oder Wasser vorgesehen ist Weiterhin ist die Absperrverteilervorrichtung (13) mit mehreren Funktionsstellungen ausgebildet, die zumindest ein Herstellen und Unterbrechen einer Strömungsverbindung zwischen dem ersten Reservoir (11) und/oder dem zweiten Reservoir (12) und dem Anschluss (26) umfassen, dadurch gekennzeichnet, wobei erstes Reservoir (11), zweites Reservoir (12) und Absperrverteilervorrichrung (13) als zumindest im Betriebszustand miteinander starr verbundene Einheit ausgebildet sind.

## Beschreibung

Die Erfindung betrifft eine Vorrichtung zur Verabreichung enteraler Ernährung umfassend ein erstes Reservoir sowie ein zweites Reservoir, die jeweils über eine Absperrverteilervorrichtung mit einem zum Patienten führenden Anschluss in Verbindung stehen, wobei das erste Reservoir zur Befüllung mit enteraler Ernährung und das zweite Reservoir zur Befüllung mit Flüssigkeit, insbesondere Tee oder Wasser vorgesehen ist, wobei die Absperrvorrichtung mit mehreren Funktionsstellungen ausgebildet ist, die zumindest ein Herstellen und Unterbrechen einer Strömungsverbindung zwischen dem ersten Reservoir und/öder dem zweiten Reservoir und dem Anschluss umfassen nach dem Oberbegriff vom Patentanspruch 1. Eine derartige Vorrichtung zum Verabreichen enteraler Ernährung ist bereits aus der EP 1 129 682 A2 bekannt.

Die Aufgabe der vorliegenden Erfindung besteht darin, eine derartige Vorrichtung in ihrer Handhabbarkeit noch zu verbessern und den Betrieb einer solchen Vorrichtung reproduzierbarer zu gestalten.

Diese Aufgabe wird mit einer Vorrichtung nach den Merkmalen des Anspruches 1 gelöst. Vorteilhafte Weiterbildungen sind in den Unteransprüchen angegeben.

Ein Kerngedanke der vorliegenden Erfindung besteht darin, erstes Reservoir, zweites Reservoir und Absperrverteilervorrichtung als zumindest im Betriebszustand miteinander starr verbundene Einheit auszubilden.

Im Gegensatz zur vorbekannten Vorrichtung ist die erfindungsgemäße Vorrichtung wesentlich kompakter aufgebaut. Der an der Absperrverteilervorrichtung anstehende Fluiddruck der enteralen Ernährung bzw. der Flüssigkeit ist reproduzierbarer, da erfindungsgemäß erstes Reservoir und zweites Reservoir im Betriebszustand mit der Absperrverteilervorrichtung starr verbunden sind. Bei der vorbekannten Vorrichtung hängt dies hingegen von der Aufhänghöhe des jeweiligen Reservoirs ab.

In der ersten alternativen Ausgestaltung sind erstes Reservoir und/oder zweites Reservoir und Absperrverteilervorrichtung einstückig zusammenhängend ausgebildet. Es ist aber auch möglich erstes Reservoir und/oder zweites Reservoir von der Absperrverteilervorrichtung, beispielsweise über einen Schraubanschluss oder über einen Einschnappanschluss lösbar auszubilden. Derartige Anschlussvarianten sind beispielsweise aus der DE 38 28 729 bekannt.

In einer besonders vorteilhaften Ausgestaltung der vorliegenden Erfindung sind erstes Reservoir und zweites Reservoir durch ein gemeinsames Flächenstück, das eine Trennwand zwischen erstem Reservoir und zweitem Reservoir ausbildet, getrennt. Erstes Reservoir und zweites Reservoir grenzen damit beide unmittelbar aneinander an, was die Kompaktheit der gesamten Vorrichtung noch erhöht.

In einer vorteilhaften Ausgestaltung umfasst die Absperrverteilervorrichtung weiterhin Mittel zur Dosierung des an den Anschluss überführten Fluidstroms. In dieser Ausgestaltung übernimmt die Absperrverteilervorrichtung nicht nur die Funktion des Herstellens bzw. Unterbrechens einer Strömungsverbindung zwischen dem ersten Reservoir und/oder dem zweiten Reservoir und dem Anschluss, sondern ist gleichzeitig in der Lage, diesen Fluidstrom, der aus Bezug von Substanz aus dem ersten Reservoir und/oder zweiten Reservoir herrührt, zu dosieren. Eine externe Dosiervorrichtung kann in dieser bevorzugten Ausgestaltung daher entfallen.

In einer weiter bevorzugten Ausgestaltung ist die Absperrverteilervorrichtung dazu ausgebildet, ein gewünschtes Mischungsverhältnis zwischen Entnahme von enteraler Ernährung aus dem Reservoir und Entnahme von Flüssigkeit aus dem zweiten Reservoir einzustellen. In dieser Ausgestaltung sind beliebige Mischung aus enteraler Ernährung und Flüssigkeit, beispielsweise Tee möglich, insbesondere um enterale Ernährung bei Ernährungsbeginn zu verdünnen. Auch überkalorische Nahrungen können stufenlos herunter verdünnt werden.

Gleichzeitig ist es möglich, zum Spülen der Sonde oder um nicht selten auftretende Verstopfungen in den bei der Überleitung an den Patienten verwendeten Sonden zu vermeiden, das Mischungsverhältnis kurzfristig auf wesentlich höhere Flüssigkeitsgabe bzw. Teegabe oder auch gänzlich auf Flüssigkeit bzw. Tee umzustellen.

In einer bevorzugten Ausgestaltung sind erstes Reservoir und zweites Reservoir jeweils mit einem Fassungsvolumen von 600 ml bis 1500 ml, bevorzugt von jeweils 1000 ml ausgebildet.

In einer weiteren Ausgestaltung umfassen erstes Reservoir und/oder zweites Reservoir an ihrer der Absperrverteilervorrichtung abgewandten Seite jeweils eine, vorzugsweise mit Deckel verschließbare Befüllöffnung, zur Nachfüllung von enteraler Ernährung bzw. von Flüssigkeit.

Enterale Ernährung kann besonders preiswert primär in Schlauchbeuteln aus Aluminiumverbundfolie nachgefüllt werden. In Schlauchbeuteln aus Aluminiumverbundfolie besteht optimaler Schutz vor Licht und Sauerstoff und damit lange Haltbarkeit, wobei ein Öffnen des Schlauchbeutels erst unmittelbar vor Verabreichung an den Patienten trotz der erfindungsgemäßen Vorrichtung notwendig ist. Das Umfüllen in das zugeordnete Reservoir der erfindungsgemäßen Vorrichtung gelingt ohne Hilfsmittel. Auch eine Nachbefüllung aus Glasflaschen ist ohne Weiteres möglich und gelingt ebenfalls ohne Hilfsmittel.

Nach einem besonders bevorzugten Aspekt der vorliegenden Erfindung bilden erstes Reservoir und zweites Reservoir ein zusammenhängendes Behältnis mit einer gemeinsamen Außenwand aus. Hierdurch wird die kompakte Bauform noch begünstigt. Es entsteht ein Gesamtbehältnis mit zwei getrennten Kompartimenten, die jeweils erstes und zweites Reservoir ausbilden.

Bevorzugtermaßen umfasst die Absperrverteilervorrichtung ein mit dem ersten Reservoir und/oder dem zweiten Reservoir einstückig verbundenes oder starr verbindbares Außenelement mit mindestens zwei Einlässen und mindestens einem Auslass sowie ein gegenüber dem Außenelement zumindest drehbewegliches Innenelement. Mit dieser Ausgestaltung lassen sich zumindest die grundsätzlichen Funktionsstellungen, nämlich Herstellen und Unterbrechen einer Strömungsverbindung zwischen dem ersten Reservoir und/oder dem zweiten Reservoir und dem Anschluss leicht realisieren. Außen- und Innenelement können aber auch so aufeinander abgestimmt sein, dass sich in definierten Rastpositionen oder kontinuierlich gewünschte Mischungsverhältnisse zwischen Entnahme von enteraler Ernährung aus dem ersten Reservoir und Entnahme von Flüssigkeit aus dem zweiten Reservoir einstellen lassen.

In einer besonders bevorzugten Weiterbildung ist das Innenelement gegenüber dem Außenelement zusätzlich translatorisch bewegbar, wobei Außenelement und Innenelement so aufeinander abgestimmt sind, dass sich durch diese translatorische Verschiebung eine gewünschte Dosierung des an den Anschluss überführten Fluidstroms einstellen lässt. In dieser Ausgestaltung lässt sich mit der Absperrverteilervorrichtung nicht nur ein gewünschtes Mischungsverhältnis zwischen enteraler Ernährung und Flüssigkeit in diskreten Schritten oder kontinuierlich einstellen, sondern es kann auch die rein aus enteraler Ernährung, rein aus Flüssigkeit oder aus einer Mischung bestehende Abgabe ebenfalls in diskreten Schritten oder kontinuierlich dosiert werden.

In einer zweckmäßigen Ausgestaltung ist weiterhin eine Aufhängvorrichtung derart ausgebildet und angeordnet, dass bei Aufhängung der Vorrichtung sich erstes Reservoir und zweites Reservoir beide oberhalb der Absperrvorrichtung befinden.

Die Erfindung wird nachstehend auch hinsichtlich weiterer Merkmale und Vorteile anhand der Beschreibung von Ausführungsbeispielen und unter Bezugnahme auf die nachstehenden Zeichnungen näher erläutert.

Hierbei zeigen:
- Fig. 1:: eine schematische Darstellung einer bevorzugten Ausführungsform der erfindungs gemäßen Vorrichtung in perspektivischer Ansicht.
- Fig. 2:: eine Explosionsdarstellung der anhand von Fig. 1 veranschaulichten Ausführungsform, ebenfalls in perspektivischer Ansicht
- Fig. 3: eine Schnittansicht der Vorrichtung aus Fig. 1 entlang der Linie III

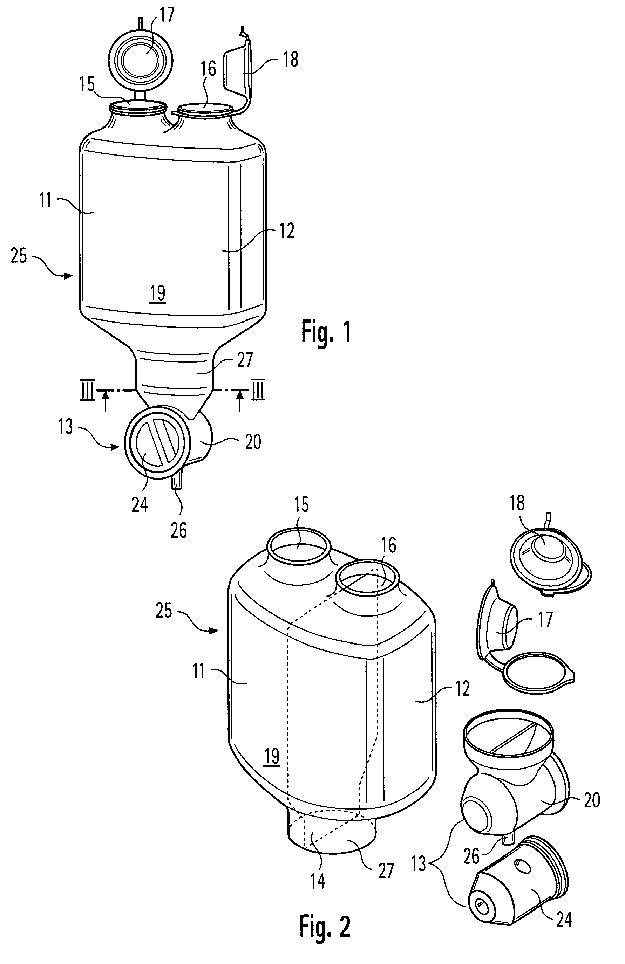

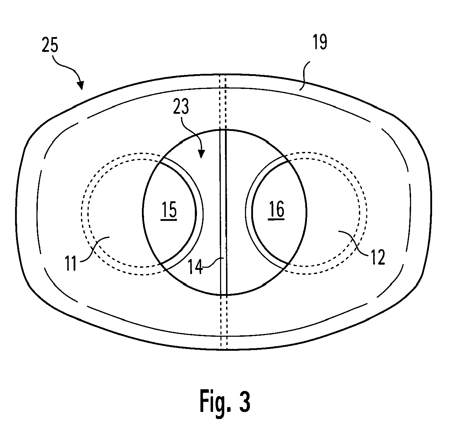

Anhand der Figuren 1 bis 3 ist eine bevorzugte Ausführungsform der erfindungsgemäßen Vorrichtung umfassend ein Behältnis 25 mit einem Überführungsabschnitt 27 sowie einer am Überführungsabschnitt 27 starr angeschlossenen Absperrverteilervorrichtung 13, veranschaulicht.. Das Behältnis 25 weist eine umlaufende Außenwand 19 auf und ist über eine Trennwand 14 in zwei - hier gleich große - Kompartimente unterteilt, die ein erstes Reservoir 11 und ein zweites Reservoir 12 ausbilden.

Erstes Reservoir 11 und zweites Reservoir 12 sind jeweils über Befüllöffnungen 15, 16 mit enteraler Ernährung bzw. Flüssigkeit, insbesondere Wasser oder Tee befüllbar. Die Befüllöffnungen 15, 16 lassen sich über Deckel 17, 18 verschließen.

Die Absperrverteilervorrichtung 13 kann mit dem Behältnis 25 dauerhaft, insbesondere über Kleben, Schweißen oder einstückige Ausbildung im Formprozess dauerhaft verbunden sein. Die Absperrverteilervorrichtung 13 umfasst ein Außenelement 20 sowie ein gegenüber dem Außenelement 20 drehbeweglich und translatorisch verschiebbar gelagertes Innenelement 24. Das Außenelement 20 umfasst dem Innenelement 24 zugewandt mindestens zwei Einlässe sowie mindestens einen Auslass.

Durch Drehen des Innenelements 24 im Außenelement 20 lassen sich die gewünschten Funktionsstellungen hinsichtlich Herstellen und Unterbrechen einer Strömungsverbindung zwischen dem ersten Reservoir 11 oder dem zweiten Reservoir 12 und einem Anschluss 26 bzw. Mischungsverhältnisse zwischen Entnahme von enteraler Ernährung aus dem ersten Reservoir 11 und Entnahme von Flüssigkeit aus dem zweiten Reservoir 12 einstellen. Durch translatorisches Verschieben des Innenelements 24 im Außenelement 20 lässt sich die an den Anschluss 26 überführte Abgabemenge dosieren. An den Anschluss 26 können an sich bekannte Überleitungssysteme angeschlossen werden oder bereits angeschlossen sein.

Die erfindungsgemäße Vorrichtung ermöglicht die Gabe von enteraler Ernährung und von Flüssigkeit, insbesondere von Tee oder Wasser, nacheinander oder in beliebigem Mischungsverhältnis sowie in der gewünschten Dosierung. Über die Dosierung lassen sich die üblicherweise geforderten Tropfarten zwischen 25 ml/h bis maximal 300ml/h einstellen. Eine Rollenbremse oder Pumpe ist bei der erfindungsgemäßen Vorrichtung verzichtbar.

Die erfindungsgemäße Vorrichtung ermöglicht auch nach der Gabe der enteralen Ernährung auf Flüssigkeit, insbesondere Tee bzw. Wasser zur Flüssigkeitsgabe oder zum Spülen der Überleitungsmimik inklusive der Sonde umzustellen.

Das Umwechseln auf ein anderes System oder die Füllung des enteralen Systems mit Tee/Wasser nach Gabe der enteralen Ernährung ist bei der erfindungsgemäßen Vorrichtung nicht notwendig. Das mindestens zwei Reservoirs 11, 12 umfassende Behältnis 25 kann preiswert mit enteraler Ernährung aus Flaschen oder Beuteln befüllt werden. Das Behältnis 25 sowie die daran befestigte oder befestigbare Absperrverteilervorrichtung 13 können ohne weiteres zehn Tage und länger für den gleichen Patienten verwendet werden. Am Ende der täglichen Anwendung erfolgt die Reinigung in einer Spülmaschine, vorzugsweise in einer medizinischen Spülmaschine (95°C).

In einer weiter bevorzugten Ausgestaltung ist das Innenelement 24 der Absperrverteilervorrichtung 13 vom Außenelement 20 lösbar oder alternativ bzw. zusätzlich die Absperrverteilervorrichtung 13 insgesamt vom Behältnis 25 lösbar, so dass es auch möglich ist, Innenelement 24 bzw. die gesamte Absperrverteilervorrichtung 13 nach einer Nutzungsdauer von einem Tag zu entfernen und zu entsorgen.

Die Kosten des Gesamtsystems können so, bei gleichzeitig bequemer Handhabung, niedrig gehalten werden.

Ein neues Innenelement 24 bzw. eine neue Absperrverteilervorrichtung 13 als Austauschteil kann ggf. mit anhängendem Überleitungssystem nach MPG sterilisiert und verpackt werden, wie dies bei herkömmlichen Überleitungsgeräten auch bislang der Fall ist.

### Bezugszeichenliste

- 11: erstes Reservoir
- 12: zweites Reservoir
- 13: Absperrverteilervorrichtung
- 14: Trennwand
- 15, 16: Befüllöffnung
- 17, 18: Deckel
- 19: Außenwand
- 20: Außenelement
- 24: Innenelement
- 25: Behältnis
- 26: Anschluss
- 27: Überführungsabschnitt

## Patentansprüche

1. Vorrichtung zur Verabreichung enteraler Ernährung umfassend ein erstes Reservoir (11) sowie ein zweites Reservoir (12), die jeweils über eine Absperrverteilervorrichtung (13) mit einem zum Patienten führenden Anschluss (26) in Verbindung stehen, wobei das erste Reservoir (11) zur Befüllung mit enteraler Ernährung und das zweite Reservoir (12) zur Befüllung mit Flüssigkeit, insbesondere Tee oder Wasser vorgesehen ist,
wobei die Absperrverteilervorrichtung (13) mit mehreren Funktionsstellungen ausgebildet ist, die zumindest ein Herstellen und Unterbrechen einer Strömungsverbindung zwischen dem ersten Reservoir (11) und/oder dem zweiten Reservoir (12) und dem Anschluss (26) umfassen,
**dadurch gekennzeichnet,**
**dass** erstes Reservoir (11), zweites Reservoir (12) und Absperrverteilervorrichtung (13) als zumindest im Betriebszustand miteinander starr verbundene Einheit ausgebildet sind.

2. Vorrichtung nach Anspruch 1,
**dadurch gekennzeichnet,**
**dass** erstes Reservoir (11) und/oder zweites Reservoir (12) und Absperrverteilervorrichtung (13) einstückig zusammenhängend ausgebildet sind.

3. Vorrichtung nach Anspruch 1,
**dadurch gekennzeichnet,**
**dass** erstes Reservoir (11) und/oder zweites Reservoir (12) von der Absperrverteilervorrichtung (13), beispielsweise über einen Schraubanschluss oder über einen Einschnappanschluss, lösbar ausgebildet sind.

4. Vorrichtung nach einem der Ansprüche 1 bis 3,
**dadurch gekennzeichnet,**
**dass** erstes Reservoir (11) und zweites Reservoir (12) durch ein gemeinsames Flächenstück, das eine Trennwand (14) zwischen erstem Reservoir (11) und zweitem Reservoir (12) ausbildet, getrennt sind.

5. Vorrichtung nach einem der Ansprüche 1 bis 4,
**dadurch gekennzeichnet,**
**dass** die Absperrverteilervorrichtung (13) Mittel zur Dosierung des an den Anschluss (26) überführten Fluidstroms umfasst.

6. Vorrichtung nach einem der Ansprüche 1 bis 5,
**dadurch gekennzeichnet,**
**dass** die Absperrverteilervorrichtung (13) dazu ausgebildet ist, ein gewünschtes Mischungsverhältnis zwischen Entnahme von enteraler Ernährung aus dem ersten Reservoir (11) und Entnahme von Flüssigkeit aus dem zweiten Reservoir (12) einzustellen.

7. Vorrichtung nach einem der Ansprüche 1 bis 6,
**dadurch gekennzeichnet,**
**dass** erstes Reservoir (11) und zweites Reservoir (12) jeweils mit einem Fassungsvolumen von 600 ml bis 1.500 ml, bevorzugt von jeweils 1.000 ml ausgebildet sind.

8. Vorrichtung nach einem der Ansprüche 1 bis 7,
**dadurch gekennzeichnet,**
**dass** erstes Reservoir (11) und/oder zweites Reservoir (12) an ihrer der Absperrverteilervorrichtung (13) abgewandten Seite jeweils eine, vorzugsweise mit Deckel (17, 18) verschließbare, Befüllöffnung (15, 16) zur Nachfüllung von enteraler Ernährung bzw. Flüssigkeit umfassen.

9. Vorrichtung nach einem der Ansprüche 1 bis 8,
**dadurch gekennzeichnet,**
**dass** erstes Reservoir (11) und zweites Reservoir (12) ein zusammenhängendes Behältnis (25) mit einer gemeinsamen Außenwand (19) ausbilden.

10. Vorrichtung nach einem der Ansprüche 1 bis 9,
**dadurch gekennzeichnet,**
**dass** die Absperrverteilervorrichtung (13) ein mit dem ersten Reservoir (11) und/oder dem zweiten Reservoir (12) einstückig verbundenes oder starr verbindbares Außenelement (20) mit mindestens zwei Einlässen und mindestens einem Auslass sowie ein gegenüber dem Außenelement (20) zumindest drehbewegliches Innenelement (24) umfasst.

11. Vorrichtung nach Anspruch 10,
**dadurch gekennzeichnet,**
**dass** das Innenelement (24) gegenüber dem Außenelement (20) zusätzlich translatorisch bewegbar ist, wodurch eine Veränderung des an den Anschluss (26) überführbaren Fluidstroms einstellbar ist.

12. Vorrichtung nach Anspruch 10 oder 11,
**dadurch gekennzeichnet,**
**dass** Außenelement (20) und Innenelement (24) so ausgebildet und aufeinander abgestimmt sind, dass für das Einstellen des Mischungsverhältnisses zwischen Entnahme aus dem ersten Reservoir (11) und Entnahme aus dem zweiten Reservoir (12) und/oder für das Einstellen des Fluidstroms/der Abgabemenge an den Anschluss (26) die Absperrverteilervorrichtung (13) eine vorbestimmte Anzahl diskreter Rastpositionen umfasst, um das Mischungsverhältnis bzw. die Abgabemenge in diskreten Schritten einstellen zu können.

13. Vorrichtung nach Anspruch 10 oder 11,
**dadurch gekennzeichnet,**
**dass** Außenelement (20) und Innenelement (24) so ausgebildet und aufeinander abgestimmt sind, dass eine kontinuierliche Einstellung des Mischungsverhältnisses zwischen Entnahme aus dem ersten Reservoir (11) und Entnahme aus dem zweiten Reservoir (12) und/oder für das Einstellen des Fluidstroms/der Abgabemenge an den Anschluss (26) vorgesehen ist.

14. Vorrichtung nach einem der Ansprüche 1 bis 13,
**dadurch gekennzeichnet,**
**dass** eine Aufhängvorrichtung derart ausgebildet und angeordnet ist, dass bei Aufhängung der Vorrichtung sich erstes Reservoir (11) und zweites Reservoir (12) beide oberhalb der Absperrverteilervorrichtung (13) befinden.
